# EUROPÄISCHE PATENTSCHRIFT

(11) **EP 2 906 528 B1**
(45) Veröffentlichungstag und Bekanntmachung des Hinweises auf die Patenterteilung: **21.09.2016**
(21) Anmeldenummer: 13774667.3
(22) Anmeldetag: 10.10.2013
(51) Int. Cl.: C07C 209/84, C07C 211/46

(54) **VERFAHREN ZUR GEWINNUNG VON REIN-ANILIN**
PROCESS FOR THE RECOVERY OF PURE ANILINE
PROCÉDÉ D'OBTENTION D'ANILINE BRUTE

(30) Priorität: 11.10.2012 EP 12188191
(43) Veröffentlichungstag der Anmeldung: 19.08.2015
(73) Patentinhaber: BASF SE, 67056 Ludwigshafen am Rhein (DE)
(72) Erfinder: VANDEWALLE, Koenraad, B-2390 Oostmalle (BE); VERCRUYSSE, Karen, B-9120 Melsele (BE); DENISSEN, Leo, B-2930 Brasschaat (BE); DEBERDT, Filip, B-2812 Muizen (BE); DE WINNE, Hendrik, 67433 Neustadt (DE); VAN DE VOORDE, Bart, B-2060 Antwerpen (BE); REISER, Michael, 67061 Ludwigshafen (DE); NETO, Samuel, North Bonifacio Taguig Metro Manila (PH)
(86) Internationale Anmeldenummer: PCT/EP2013/071197
(87) Internationale Veröffentlichungsnummer: WO 2014/057053

(56) Entgegenhaltungen:
- EP-A1- 1 845 079
- WO-A1-2012/052407
- JP-A- 2005 350 388

## Beschreibung

Die Erfindung betrifft ein Verfahren zur Gewinnung von Rein-Anilin durch katalytische Hydrierung von Nitrobenzol und Aufarbeitung des hierbei erhaltenen Reaktionsgemisches.

Anilin wird großtechnisch überwiegend ausgehend von Nitrobenzol durch katalytische Hydrierung erhalten. Die heterogen katalysierte Hydrierung kann in der Flüssig- oder in der Gasphase erfolgen. Gasphasenhydrierungen im Festbett sind beispielsweise in US 4740621 oder EP 748790 offenbart. Eine Gasphasenhydrierung im Wirbelbett ist in US 3,136,818 offenbart, die Nitrobenzolhydrierung in der Flüssigphase beispielsweise in US 4,415,754 oder US 3,270,057.

Der Reaktionsaustrag der heterogen katalysierten Hydrierung in der Flüssig- oder Gasphase enthält Anilin, Wasserstoff, Wasser sowie Nebenprodukte, beispielsweise Phenole und Nitrophenole, je nach Prozessführung auch Spuren von nicht-reagiertem Nitrobenzol.

Im Falle der Gasphasenhydrierung wird eine ein- oder mehrstufige partielle Kondensation des Reaktionsaustrages mit oder ohne Wärmeintegration durchgeführt.

Daran schließt sich eine Gas/Flüssig-Trennung mit Kondensation von mehr als 95 % von Reaktionswasser und Anilin und Abtrennung der wasserstoffreichen Gasphase an.

Die hierbei erhaltene Flüssigphase wird einer ein- oder mehrstufigen Flüssig/Flüssig-Trennung zugeführt, unter Erhalt von Roh-Anilin als organischer Phase, das anschließend einer destillativen Vorreinigung zur teilweisen oder vollständigen Abtrennung des darin enthaltenen Wassers über den Kopfstrom einer ersten Destillationskolonne zugeführt wird, aus der ein Sumpfstrom erhalten wird, der einer Rein-Destillation in einer Rein-Kolonne zugeführt wird, aus der über Kopf Rein-Anilin abgezogen wird sowie ein Sumpfstrom, der neben Anilin Schwersieder enthält, der ausgeschleust und einer Verbrennung zugeführt wird. Hierfür muss dieser Ausschleusungsstrom über Rohrleitungen förderbar sein.

Es ist anzustreben, das obige Verfahren, insbesondere auch den letzten Verfahrensschritt, die destillative Auftrennung in der Reinkolonne, dergestalt zu betreiben, dass die Verluste an Wertprodukt Anilin möglichst niedrig sind.

Problematisch ist jedoch, dass sich die Förderbarkeit des Ausschleusungsstromes aus der Reinkolonne mit zunehmender Abreicherung an Anilin verschlechtert, bis hin zur vollständigen Blockierung der Rohrleitungen aufgrund von Erstarren der hochsiedenden Komponenten. Daher wird in großtechnischen Anlagen bislang ein Anteil von etwa 1 %, bezogen auf das Gewicht des Feed-Stromes zur Reinkolonne als Sumpfstrom aus derselben ausgeschleust. Dieser Sumpfstrom enthält dann noch etwa 60 Gew.-% Anilin, Rest Schwersieder gegenüber Anilin, und weist einen Erstarrungspunkt von etwa 90°C auf, so dass er mit einer entsprechend ausgelegten Begleitbeheizung fließfähig gehalten wird und somit durch die Rohrleitungen zur Verbrennung förderbar ist. Die Installation einer solchen Begleitbeheizung, insbesondere als Doppelmantel oder als Begleitheizungsrohre ausgebildet, ist stets deutlich teurer als isolierte oder unisolierte Rohrleitungen speziell bei großen zu überwindenden Distanzen. Darüber hinaus besteht weiterhin die Gefahr der Verblockung von Rohrleitungen, falls die Begleitbeheizung ausfällt.

Die JP 2005 350388 A beschreibt ein Verfahren zur Herstellung von Anilin durch katalytische Hydrierung von Nitrobenzol und Aufarbeitung des hierbei erhaltenen Reaktionsgemisches, wobei die Anilin-Verluste durch Zugabe von Hochsiedern begrenzt werden sollen.

Das Dokument beschreibt nicht den Zusatz von Methanol zum Sumpfstrom aus der Anilin-Reinkolonne.

Es war demgegenüber Aufgabe der Erfindung, ein einfaches, zuverlässiges und kostengünstiges Verfahren zur Verfügung zu stellen, wonach die Verluste an Wertprodukt Anilin über den Ausschleusungsstrom aus der Reinkolonne begrenzt werden können.

Diese Aufgabe wird gelöst durch ein Verfahren zur Gewinnung von Rein-Anilin mit den folgenden Verfahrensschritten
- katalytische Hydrierung von Nitrobenzol,
- optional partielle Kondensation des Reaktionsgemisches der katalytischen Hydrierung in einer oder mehreren Stufen,
- Auftrennung des nach der katalytischen Hydrierung oder der optional durchgeführten partiellen Kondensation erhaltenen Reaktionsgemisches in eine Wasserstoff enthaltende Gasphase und eine Flüssigphase und
- Flüssig/Flüssig-Phasentrennung der hierbei erhaltenen Flüssigphase, unter Erhalt einer wässrigen Phase, die einer weiteren Aufarbeitung zugeführt oder ausgeschleust wird, sowie von Roh-Anilin als organische Phase, wobei das Roh-Anilin 90-95 Gew.-% Anilin, 4-9 Gew.-% Wasser und Rest Schwersieder gegenüber Anilin, jeweils bezogen auf das Gesamtgewicht des Roh-Anilins, enthält, und wobei die Summe der Komponenten des Roh-Anilins 100 Gew.-% beträgt,
- destillative Vorreinigung des Roh-Anilins durch teilweise oder vollständige Abtrennung des Wassers über den Kopfstrom einer ersten Destillationskolonne unter Erhalt eines Sumpfstromes, der als Feed-Strom
- einer Reinkolonne zugeführt wird, aus der über Kopf ein Rein-Anilin-Strom, enthaltend mindesten 99,9 Gew.-% Anilin, bezogen auf das Gesamtgewicht des Rein-Anilin-Stromes, sowie ein Sumpfstrom, enthaltend Schwersieder und der einer Verbrennung zugeführt wird, abgezogen werden,
das dadurch gekennzeichnet ist, dass der Gewichtsanteil des Ausschleusungsstromes aus der Reinkolonne auf maximal 0,8 %, bezogen auf das Gewicht des Feed-Stromes begrenzt wird, und gewährleistet wird, dass der Ausschleusungsstrom unter entsprechender Begleitbeheizung der hierfür vorgesehenen Leitungen, die gewährleistet, dass die Temperatur des darin geförderten Stromes 45 °C oder höher ist, pumpfähig ist, indem dem Sumpfstrom technisches Methanol, Ethanol, Propanol, Aceton oder Mischungen hiervon in einem Gewichtsanteil von 5-30 %, bezogen auf das Gewicht des Sumpfstromes, zugesetzt wird.

Roh-Anilin kann zusätzlich unmittelbar bei der partiellen Kondensation des Reaktionsgemisches der katalytischen Hydrierung in einer oder mehreren der einen oder mehreren Stufen der partiellen Kondensation anfallen.

Bevorzugt wird dem Sumpfstrom Methanol, Ethanol, Propanol, Aceton oder Mischungen hiervon in einem Gewichtsanteil von 10 bis 30 %, bezogen auf das Gewicht des Sumpfstromes, zugesetzt.

In einer bevorzugten Ausführungsform umfasst die Erfindung ein Verfahren zur Gewinnung von Rein-Anilin mit den folgenden Verfahrensschritten
- katalytische Hydrierung von Nitrobenzol,
- Kondensation des Reaktionsgemisches der katalytischen Hydrierung
- Flüssig/Flüssig-Phasentrennung des hierbei gebildeten Kondensats, unter Erhalt einer wässrigen Phase, die einer weiteren Aufarbeitung zugeführt oder ausgeschleust wird, sowie von Roh-Anilin als organische Phase, wobei das Roh-Anilin 90-95 Gew.-% Anilin, 4-9 Gew.-% Wasser und Rest Schwersieder gegenüber Anilin, jeweils bezogen auf das Gesamtgewicht des Roh-Anilins, enthält, und wobei die Summe der Komponenten des Roh-Anilins 100 Gew.-% beträgt,
- destillative Vorreinigung des Roh-Anilins durch teilweise oder vollständige Abtrennung des Wassers über den Kopfstrom einer ersten Destillationskolonne unter Erhalt eines Sumpfstromes, der als Feed-Strom
- einer Reinkolonne zugeführt wird, aus der über Kopf ein Rein-Anilin-Strom, enthaltend mindesten 99,9 Gew.-% Anilin, bezogen auf das Gesamtgewicht des Rein-Anilin-Stromes, sowie ein Sumpfstrom, enthaltend Schwersieder und der einer Verbrennung zugeführt wird, abgezogen werden,
das dadurch gekennzeichnet ist, dass der Gewichtsanteil des Sumpfstromes aus der Reinkolonne auf maximal 0,8 %, bezogen auf das Gewicht des Feed-Stromes begrenzt wird, und dadurch gewährleistet wird, dass derselbe keine festen Anteile enthält und der somit, unter entsprechender Begleitbeheizung der hierfür vorgesehenen Leitungen, pumpfähig ist, indem dem Sumpfstrom technisches Methanol, in einem Gewichtsanteil von 15-30 %, bezogen auf das Gewicht des Sumpfstromes, zugesetzt wird.

Es wurde gefunden, dass es in einfacher Weise möglich ist, den Verlust an Wertprodukt Anilin über den Sumpfstrom aus der Reinkolonne weiter zu reduzieren, und gleichzeitig die Pumpfähigkeit desselben zu gewährleisten, indem demselben Methanol, Ethanol, Propanol, Aceton oder Mischungen hiervon zugesetzt werden.

Als Roh-Anilin-Strom wird vorliegend ein Strom verstanden, der 90 bis 95 Gew.-% Anilin, 4 bis 9 Gew.-% Wasser und Rest Schwersieder gegenüber Anilin, jeweils bezogen auf das Gesamtgewicht des Roh-Anilins, enthält, und wobei die Summe der Komponenten des Roh-Anilins 100 Gew.-% ergibt, oder auch ein Strom, der 93 bis 94 Gew.-% Anilin, 5 bis 6 Gew.-% Wasser und Rest Schwersieder gegenüber Anilin enthält.

Als Rein-Anilin-Strom wird vorliegend ein Strom verstanden, der mindestens 99,7 Gew.-% Anilin oder auch mindestens 99,9 Gew.-% Anilin, Rest Verunreinigungen, enthält.

Als technisches Methanol wird vorliegend ein Strom verstanden, mindestens 95 Gew.-% Methanol oder auch mindestens 99 Gew.-% Methanol, Rest Verunreinigungen, enthält.

Durch Zusatz von Methanol, Ethanol, Propanol, Aceton oder Mischungen hiervon in einem Gewichtsanteil im Bereich von 5-30 %, bezogen auf das Gewicht des Sumpfstromes, kann derselbe an Wertprodukt Anilin abgereichert und dennoch pumpbar bleiben. Hierfür ist eine Begleitbeheizung für die den Ausschleusungsstrom aus der Reinkolonne führenden Leitungen vorzusehen, die derart betrieben wird, dass die Temperatur des darin geförderten Stromes 45°C oder höher ist.

Bevorzugt wird technisches Methanol dem Sumpfstrom in einem Gewichtsanteil von 20-25 %, bezogen auf das Gewicht des Sumpfstromes, zugesetzt.

Bevorzugt wird die Begleitbeheizung der Gestalt ausgelegt, dass der Ausschleusungsstrom, der in den begleitbeheizten Leitungen gefördert wird, eine Temperatur von 60°C oder höher aufweist und pumpfähig bleibt.

Bevorzugt wird technisches Methanol unter einem Überdruck von 15-20 bar zugeführt; dies ist vorteilhaft, da die Verbrennung üblicherweise bei Überdruck stattfindet und somit zu verbrennende Ströme unter erhöhtem Druck relativ zum Brennerdruck zugeführt werden müssen.

Das erfindungsgemäße Verfahren erlaubt es, den Betriebspunkt der Anilin-Rein-Kolonne so einzustellen, dass der Gewichtsanteil des Sumpfstroms, der aus der Reinkolonne ausgeschleust wird, bezogen auf das Gewicht des Feed-Stromes zu derselben, von derzeit etwa 1 %, auf maximal 0,8 %, oder auch auf maximal 0,7 %, desselben reduziert werden kann, und dabei gleichzeitig gewährleistet ist, dass der Sumpfstrom pumpfähig bleibt.

Bei einem Anteil von 1 % des Ausschleusungsstromes, bezogen auf den Feed-Strom enthält der Ausschleusungsstrom noch etwa 60 Gew.-% Anilin, Rest Schwersieder gegenüber Anilin.

Der Gewichtsanteil des Sumpfstromes aus der Reinkolonne wird erfindungsgemäß auf 0,8 % des Feedstromes zu derselben begrenzt. Dann enthält derselbe noch etwa 40 Gew.-% Anilin, Rest Schwersieder.

Durch die Begrenzung des Sumpfstromes auf maximal 0,8 % des Feedstromes nach dem erfindungsgemäßen Verfahren gegenüber 1 % nach dem Stand der Technik wird bei einer Produktion von beispielsweise 1.000.000 t eine Einsparung von 2.000 t Anilin pro Jahr erreicht.

Die Erfindung wird im Folgenden anhand eines Ausführungsbeispiels erläutert:
Der bei Raumtemperatur feste Sumpfaustrag aus einer großtechnischen Anilin-Reindestillationskolonne entsprechend der US 3,136,818 wurde bei 90°C homogenisiert und in ein 200 ml Glasdruckgefäß gefüllt.

Anschließend wurde bei in der nachfolgend aufgeführten, definierten Temperaturen so viel 99,8 Gew.-%iges Methanol (technisches Methanol) zudosiert, bis alle Feststoffanteile gelöst waren. Die hierfür nötigen Methanolmengen sind in der nachfolgenden Tabelle angegeben:

| **Temperatur [°C]** | **Gew.% zudosiertes 99,8 Gew.-%iges Methanol** |
|---|---|
| 100 | 0 |
| 90 | 0 |
| 80 | 4,1 |
| 60 | 15,0 |
| 50 | 22,0 |
| 40 | 34,0 |

Die obigen Versuche zeigen, dass durch Zudosieren von 99,8 Gew.-%igem Methanol in einem Gewichtsanteil im beanspruchten Bereich von etwa 5-30 Gew.-%, bezogen auf das Gewicht des Sumpfstromes, alle Feststoffanteile aus demselben bei einer Temperatur von größer als etwa 45°C bis etwas unterhalb 80°C, gelöst waren.

## Patentansprüche

1. Verfahren zur Gewinnung von Rein-Anilin mit den folgenden Verfahrensschritten
- katalytische Hydrierung von Nitrobenzol,
- optional partielle Kondensation des Reaktionsgemisches der katalytischen Hydrierung in einer oder mehreren Stufen,
- Auftrennung des nach der katalytischen Hydrierung oder der optional durchgeführten partiellen Kondensation erhaltenen Reaktionsgemisches in eine Wasserstoff enthaltende Gasphase und eine Flüssigphase und
- Flüssig/Flüssig-Phasentrennung der hierbei erhaltenen Flüssigphase, unter Erhalt einer wässrigen Phase, die einer weiteren Aufarbeitung zugeführt oder ausgeschleust wird, sowie von Roh-Anilin als organische Phase, wobei das Roh-Anilin 90-95 Gew.-% Anilin, 4-9 Gew.-% Wasser und Rest Schwersieder gegenüber Anilin, jeweils bezogen auf das Gesamtgewicht des Roh-Anilins, enthält, und wobei die Summe der Komponenten des Roh-Anilins 100 Gew.-% beträgt,
- destillative Vorreinigung des Roh-Anilins durch teilweise oder vollständige Abtrennung des Wassers über den Kopfstrom einer ersten Destillationskolonne unter Erhalt eines Sumpfstromes, der als Feed-Strom
- einer Reinkolonne zugeführt wird, aus der über Kopf ein Rein-Anilin-Strom, enthaltend mindesten 99,9 Gew.-% Anilin, bezogen auf das Gesamtgewicht des Rein-Anilin-Stromes, sowie ein Sumpfstrom, enthaltend Schwersieder und der einer Verbrennung zugeführt wird, abgezogen werden,
**dadurch gekennzeichnet, dass** der Gewichtsanteil des Sumpfstromes aus der Reinkolonne auf maximal 0,8 %, bezogen auf das Gewicht des Feed-Stromes begrenzt wird, und dadurch gewährleistet wird, dass derselbe unter entsprechender Begleitbeheizung der hierfür vorgesehenen Leitungen, die gewährleistet, dass die Temperatur des darin geförderten Stromes 45°C oder höher ist, pumpfähig ist, indem dem Sumpfstrom Methanol, Ethanol, Propanol, Aceton oder Mischungen hiervon in einem Gewichtsanteil von 5-30 %, bezogen auf das Gewicht des Sumpfstromes, zugesetzt wird.

2. Verfahren nach Anspruch 1, **dadurch gekennzeichnet, dass** Roh-Anilin zusätzlich unmittelbar bei der partiellen Kondensation des Reaktionsgemisches der katalytischen Hydrierung in einer oder mehreren der einen oder mehreren Stufen der partiellen Kondensation anfällt.

3. Verfahren nach Anspruch 1 oder 2, **dadurch gekennzeichnet, dass** dem Sumpfstrom Methanol, Ethanol, Propanol, Aceton oder Mischungen hiervon in einem Gewichtsanteil von 10 bis 30 %, bezogen auf das Gewicht des Sumpfstromes, zugesetzt wird.

4. Verfahren zur Gewinnung von Rein-Anilin nach Anspruch 1 mit den folgenden Verfahrensschritten
- katalytische Hydrierung von Nitrobenzol,
- Kondensation des Reaktionsgemisches der katalytischen Hydrierung
- Flüssig/Flüssig-Phasentrennung des hierbei gebildeten Kondensats, unter Erhalt einer wässrigen Phase, die einer weiteren Aufarbeitung zugeführt oder ausgeschleust wird, sowie von Roh-Anilin als organische Phase, wobei das Roh-Anilin 90-95 Gew.-% Anilin, 4-9 Gew.-% Wasser und Rest Schwersieder gegenüber Anilin, jeweils bezogen auf das Gesamtgewicht des Roh-Anilins, enthält, und wobei die Summe der Komponenten des Roh-Anilins 100 Gew.-% beträgt,
- destillative Vorreinigung des Roh-Anilins durch teilweise oder vollständige Abtrennung des Wassers über den Kopfstrom einer ersten Destillationskolonne unter Erhalt eines Sumpfstromes, der als Feed-Strom
- einer Reinkolonne zugeführt wird, aus der über Kopf ein Rein-Anilin-Strom, enthaltend mindestens 99,9 Gew.-% Anilin, bezogen auf das Gesamtgewicht des Rein-Anilin-Stromes, sowie ein Sumpfstrom, enthaltend Schwersieder und der einer Verbrennung zugeführt wird, abgezogen werden,
**dadurch gekennzeichnet, dass** der Gewichtsanteil des Sumpfstromes aus der Reinkolonne auf maximal 0,8 %, bezogen auf das Gewicht des Feed-Stromes begrenzt wird, und dadurch gewährleistet wird, dass derselbe keine festen Anteile enthält und der somit, unter entsprechender Begleitbeheizung der hierfür vorgesehenen Leitungen, pumpfähig ist, indem dem Sumpfstrom technisches Methanol, in einem Gewichtsanteil von 15-30 %, bezogen auf das Gewicht des Sumpfstromes, zugesetzt wird.

5. Verfahren nach einem der Ansprüche 1 bis 4, **dadurch gekennzeichnet, dass** der Gewichtsanteil des Sumpfstromes aus der Reinkolonne auf maximal 0,7 % des Feed-Stromes zu derselben, begrenzt wird.

6. Verfahren nach Anspruch 4 oder 5, **dadurch gekennzeichnet, dass** dem Sumpfstrom technisches Methanol in einem Gewichtsanteil von 20-25 %, bezogen auf das Gewicht des Sumpfstromes, zugesetzt wird.

7. Verfahren nach einem der Ansprüche 1 bis 6, **dadurch gekennzeichnet, dass** die Begleitbeheizung der für den Sumpfstrom aus der Reinkolonne vorgesehenen Leitungen eine Temperatur der darin geförderten Flüssigkeit von 60 °C oder höher gewährleistet.

8. Verfahren nach einem der Ansprüche 4 bis 7, **dadurch gekennzeichnet, dass** das technische Methanol dem Sumpfstrom aus der Reinkolonne unter einem Überdruck von 15 bis 20 bar zugeführt wird.

## Claims

1. A process for obtaining pure aniline, which comprises the following process steps
- catalytic hydrogenation of nitrobenzene,
- optionally partial condensation of the reaction mixture from the catalytic hydrogenation in one or more stages,
- separation of the reaction mixture obtained after the catalytic hydrogenation or the optional partial condensation into a hydrogen-comprising gas phase and a liquid phase and
- liquid/liquid phase separation of the liquid phase formed to give an aqueous phase which is passed to a further work-up or discharged and also crude aniline as organic phase, where the crude aniline comprises 90-95% by weight of aniline, 4-9% by weight of water and balance components having boiling points higher than that of aniline, in each case based on the total weight of the crude aniline, and the sum of the components of the crude aniline is 100% by weight,
- prepurification by distillation of the crude aniline by partial or complete removal of the water via the overhead stream of a first distillation column to give a bottom stream which is fed as feed stream
- to a pure column from which a pure aniline stream comprising at least 99.9% by weight of aniline, based on the total weight of the pure aniline stream, is taken off at the top and a bottom stream comprising high boilers is taken off and passed to incineration,
wherein the proportion by weight of the bottom stream from the pure column is limited to not more than 0.8%, based on the weight of the feed stream, and it is ensured that this stream is pumpable with appropriate accompanying heating of the lines provided for this purpose, which accompanying heating ensures that the temperature of the stream conveyed in said lines is 45°C or above, by adding methanol, ethanol, propanol, acetone or a mixture thereof to the bottom stream in a proportion by weight of 5-30%, based on the weight of the bottom stream.

2. The process according to claim 1, wherein crude aniline is additionally obtained directly in the partial condensation of the reaction mixture from the catalytic hydrogenation in one or more of the one or more stages of the partial condensation.

3. The process according to claim 1 or 2, wherein methanol, ethanol, propanol, acetone or a mixture thereof is added to the bottom stream in a proportion by weight of 10 to 30%, based on the weight of the bottom stream.

4. A process for obtaining pure aniline according to claim 1, which comprises the following process steps
- catalytic hydrogenation of nitrobenzene,
- condensation of the reaction mixture from the catalytic hydrogenation,
- liquid/liquid phase separation of the condensate formed to give an aqueous phase which is passed to a further work-up or discharged and also crude aniline as organic phase, where the crude aniline comprises 90-95% by weight of aniline, 4-9% by weight of water and balance components having boiling points higher than that of aniline, in each case based on the total weight of the crude aniline, and the sum of the components of the crude aniline is 100% by weight,
- prepurification by distillation of the crude aniline by partial or complete removal of the water via the overhead stream of a first distillation column to give a bottom stream which is fed as feed stream
- to a pure column from which a pure aniline stream comprising at least 99.9% by weight of aniline, based on the total weight of the pure aniline stream, is taken off at the top and a bottom stream comprising high boilers is taken off and passed to incineration,
wherein the proportion by weight of the bottom stream from the pure column is limited to not more than 0.8%, based on the weight of the feed stream, and it is ensured that this stream does not comprise any solid components and is therefore pumpable with appropriate accompanying heating of the lines provided for this purpose by adding technical-grade methanol to the bottom stream in a proportion by weight of 15-30%, based on the weight of the bottom stream.

5. The process according to any of claims 1 to 4, wherein the proportion by weight of the bottom stream from the pure column is limited to not more than 0.7% of the feed stream to this column.

6. The process according to claim 4 or 5, wherein technical-grade methanol is added to the bottom stream in a proportion by weight of 20-25%, based on the weight of the bottom stream.

7. The process according to any of claims 1 to 6, wherein the accompanying heating of the lines provided for the bottom stream from the pure column ensures a temperature of the liquid conveyed therein of 60°C or above.

8. The process according to any of claims 4 to 7, wherein the technical-grade methanol is introduced into the bottom stream from the pure column under a gauge pressure of from 15 to 20 bar.

## Revendications

1. Procédé d'obtention d'aniline pure comprenant les étapes de procédé suivantes:
- l'hydrogénation catalytique de nitrobenzène,
- éventuellement la condensation partielle du mélange réactionnel de l'hydrogénation catalytique en une ou plusieurs étapes,
- la séparation du mélange réactionnel obtenu après l'hydrogénation catalytique ou la condensation partielle éventuellement réalisée en une phase gazeuse contenant de l'hydrogène et une phase liquide, et
- la séparation de phases liquide/liquide de la phase liquide obtenue, avec obtention d'une phase aqueuse, qui est introduite dans un traitement supplémentaire ou déchargée, ainsi que d'aniline brute en tant que phase organique, l'aniline brute contenant 90 à 95 % en poids d'aniline, 4 à 9 % en poids d'eau, le reste étant des composants de point d'ébullition élevé par rapport à l'aniline, à chaque fois par rapport au poids total de l'aniline brute, et la somme des composants de l'aniline brute étant de 100 % en poids,
- la pré-purification par distillation de l'aniline brute par séparation partielle ou totale de l'eau par le courant de tête d'une première colonne de distillation, avec obtention d'un courant de fond,
- qui est introduit en tant que courant d'alimentation dans une colonne de purification, de laquelle un courant d'aniline pure est soutiré par la tête, contenant au moins 99,9 % en poids d'aniline, par rapport au poids total du courant d'aniline pure, ainsi qu'un courant de fond, contenant des composants de point d'ébullition élevé et qui est introduit dans une combustion,
**caractérisé en ce que** la proportion en poids du courant de fond de la colonne de purification est limitée à au plus 0,8 %, par rapport au poids du courant d'alimentation, ce qui assure que celui-ci soit pompable avec chauffage par traçage approprié des conduites prévues pour celui-ci, qui assure que la température du courant transporté dans celles-ci soit de 45 °C ou plus, par ajout au courant de fond de méthanol, d'éthanol, de propanol, d'acétone ou leurs mélanges en une proportion en poids de 5 à 30 %, par rapport au poids du courant de fond.

2. Procédé selon la revendication 1, **caractérisé en ce que** de l'aniline brute se forme en outre directement lors de la condensation partielle du mélange réactionnel de l'hydrogénation catalytique lors d'une ou de plusieurs de la ou des étapes de la condensation partielle.

3. Procédé selon la revendication 1 ou 2, **caractérisé en ce que** du méthanol, de l'éthanol, du propanol, de l'acétone ou leurs mélanges sont ajoutés au courant de fond en une proportion en poids de 10 à 30 %, par rapport au poids du courant de fond.

4. Procédé d'obtention d'aniline pure selon la revendication 1, comprenant les étapes de procédé suivantes:
- l'hydrogénation catalytique de nitrobenzène,
- la condensation du mélange réactionnel de l'hydrogénation catalytique,
- la séparation de phases liquide/liquide du condensat formé, avec obtention d'une phase aqueuse, qui est introduite dans un traitement supplémentaire ou déchargée, ainsi que d'aniline brute en tant que phase organique, l'aniline brute contenant 90 à 95 % en poids d'aniline, 4 à 9 % en poids d'eau, le reste étant des composants de point d'ébullition élevé par rapport à l'aniline, à chaque fois par rapport au poids total de l'aniline brute, et la somme des composants de l'aniline brute étant de 100 % en poids,
- la pré-purification par distillation de l'aniline brute par séparation partielle ou totale de l'eau par le courant de tête d'une première colonne de distillation, avec obtention d'un courant de fond,
- qui est introduit en tant que courant d'alimentation dans une colonne de purification, de laquelle un courant d'aniline pure est soutiré par la tête, contenant au moins 99,9 % en poids d'aniline, par rapport au poids total du courant d'aniline pure, ainsi qu'un courant de fond, contenant des composants de point d'ébullition élevé et qui est introduit dans une combustion,
**caractérisé en ce que** la proportion en poids du courant de fond de la colonne de purification est limitée à au plus 0,8 %, par rapport au poids du courant d'alimentation, ce qui assure que celui-ci ne contienne pas de fractions solides et soit par conséquent pompable avec chauffage par traçage approprié des conduites prévues pour celui-ci, par ajout au courant de fond de méthanol technique en une proportion en poids de 15 à 30 %, par rapport au poids du courant de fond.

5. Procédé selon l'une quelconque des revendications 1 à 4, **caractérisé en ce que** la proportion en poids du courant de fond de la colonne de purification est limitée à au plus 0,7 % du courant d'alimentation de celle-ci.

6. Procédé selon la revendication 4 ou 5, **caractérisé en ce que** du méthanol technique est ajouté au courant de fond en une proportion en poids de 20 à 25 %, par rapport au poids du courant de fond.

7. Procédé selon l'une quelconque des revendications 1 à 6, **caractérisé en ce que** le chauffage par traçage des conduites prévues pour le courant de fond de la colonne de purification assure une température du liquide transporté dans celles-ci de 60°C ou plus.

8. Procédé selon l'une quelconque des revendications 4 à 7, **caractérisé en ce que** le méthanol technique est introduit dans le courant de fond de la colonne de purification sous une surpression de 15 à 20 bar.
